# EUROPEAN PATENT SPECIFICATION

(11) **EP 0 585 215 B1**
(45) Date of publication and mention of the grant of the patent: **15.09.1999**
(21) Application number: 90917069.8
(22) Date of filing: 16.11.1990
(51) Int. Cl.: C12N 1/20, A01N 63/00, C12R 1/07

(54) **MUTANTS OR VARIANTS OF BACILLUS THURINGIENSIS PRODUCING HIGH YIELDS OF DELTA ENDOTOXIN**
MUTANTEN ODER VARIANTEN VON BACILLUS THURINGIENSIS DIE HOHE AUSBEUTEN AN DELTA ENDOTOXIN PRODUZIEREN
MUTANTS OU VARIANTS DE BACILLUS THURINGIENSIS PRODUISANT DES QUANTITES ELEVEES D'ENDOTOXINE DELTA

(30) Priority: 17.11.1989 DK 580589; 12.12.1989 DK 627489
(43) Date of publication of application: 09.03.1994
(73) Proprietor: ABBOTT LABORATORIES, Abbott Park, Illinois 60064-3500 (US)
(72) Inventor: GURTLER, Hanne, DK-2840 Holte (DK); PETERSEN, Anette, Schousbo, DK-3460 Birkerod (DK)
(74) Representative: Modiano, Guido, Dr.-Ing.
(86) International application number: DK9000294
(87) International publication number: WO9107481

(56) References cited:
- EP-A- 0 228 228
- EP-A- 0 278 035
- EP-A- 0 325 037
- EP-A- 0 328 383
- EP-A- 0 330 342
- EP-A- 0 366 398
- DE-A- 3 346 138
- US-A- 4 764 372
- CHEMICAL ABSTRACTS, Volume 80, No. 3, 21 January 1974 (Columbus, Ohio, US), DULMAGE, HOWARD T. et al.: "HD-187, a new isolate of Bacillus thuringiensis that produced high yields of alpha-endotoxin", see page 323, Abstract 13629p, & J. Invertebr. Pathol. 1973, 22(2), 273-277.
- Applied and Environmental Microbiology, Vol. 43, No. 6, June 1982, YOSHIHARU WAKISAKA et al.: "Asporogenous Bacillus thuringiensis Mutant Producing High Yields of alpha-Endotoxin", see page 1498, right column, lines 1-4.

## Description

### BACKGROUND OF THE INVENTION

Commercial preparations of Bacillus thuringiensis are used worldwide for biological control of pest insects. The advantages of these bacterial insecticides are that they are highly selective for a very limited range of target insects and are biodegradable.

Commercial preparations of Bacillus thuringiensis can be applied right up to the time of harvest with no adverse effects.

Bacillus thuringiensis is a rodshaped, aerobic, spore forming bacterium uniquely characterized by the production during the sporulation process of one or more inclusions, referred to as parasporal crystals. These crystals are composed of high molecular weight proteins, referred to as delta-endotoxins. The delta-endotoxins are the active ingredient in available commercial preparations of Bacillus thuringiensis.

Many B. thuringiensis strains with different insect host spectra have been identified. They are classified into different subspecies based on their flagellar antigens. Of particular interest is Bacillus thuringiensis subspecies kurstaki and subspecies aizawai used for the control of lepidopteran pest insects, Bacillus thuringiensis subspecies israelensis used for the control of dipteran pest insects and Bacillus thuringiensis subspecies tenebrionis used for the control of coleopteran pest insects.

The first isolation of a coleopteran toxic Bacillus thuringiensis was reported in 1983 (A. Krieg et al., Z.and.Ent. 96, 500-508, European Patent Publication EP 0149162 A2).

The isolate, which was designated Bacillus thuringiensis subsp. tenebrionis, has been deposited with the German Collection of Microorganisms under accession number DSM 2803. Bacillus thuringiensis subsp. tenebrionis was isolated in 1982 from a dead pupa of the mealworm, Tenebrio u (Tenebrionidae, Coleoptera). The strain produces within each cell one spore and one or more insecticidal parasporal crystals which are of flat platelike form with an edge length of about 0.8 µm to 1.5 µm. It belongs to serotype H8a,8b and pathotype C of Bacillus thuringiensis (Krieg et al., System.Appl.Microbiol. 9, 138-141, 1987, US patent 4,766,203, 1988).

It is only toxic against certain leaf eating beetle larvae (Chrysomelidae), but ineffective against caterpillars (Lepidoptera), mosquitoes (Diptera) or other insects.

Bacillus thuringiensis subsp. tenebrionis has been shown to be an effective control agent for the colorado potato beetle larvae. After uptake of crystals and spores from Bacillus thuringiensis subsp. tenebrionis or isolated crystals larvae, and to a certain extent adults, of the colorado potato beetle (Leptinotarsa decemlineata) stop feeding. Larval stages L1-L3 die within 1-3 days (Schnetter et al., in "Fundamental & applied aspects of invertebrate pathology", eds. R.A. Samson et al., Proceedings of the 4th Int. colloquium of Invertebrate Pathology, p. 555, 1986).

It has recently been shown that Bacillus thuringiensis subsp. tenebrionis in addition to the coleopteran active crystal also produces another parasporal crystal that is spindle-, speroidal or plateshaped (A.M. Huger & A. Krieg, J.Appl.Ent. 108, 490-497, 1989). The activity of the second crystal is not yet known.

Four commercial products of Bacillus thuringiensis subsp. tenebrionis have been developed for the control of coleopteran pests. NOVODOR® from Novo Nordisk A/S, TRIDENT® from Sandoz, and DiTerra® from Abbott Laboratories Inc., and Foil® from Ecogen.

The isolation of another coleopteran toxic Bacillus thuringiensis strain was reported in 1986 (Hernnstadt et al. Bio/Technology vol. 4, 305-308, 1986, US patent 4,764,372, 1988). This strain, designated "Bacillus thuringiensis subsp. san diego", M7, has been deposited at the Northern Regional Research Laboratory, USA under accession number NRRL B-15939. A commercial product based on "Bacillus thuringiensis subsp. san diego" has been developed by Mycogen Corp.

Comparative studies of Bacillus thuringiensis subsp. tenebrionis, DSM 2803 and "Bacillus thuringiensis subsp. san diego", NRRL-B 15939 including phenotypic characterization of the vegetative cells, characterization of the toxic parasporal crystal and analysis of plasmid DNA have, however, shown that "Bacillus thuringiensis subsp. san diego" apparently is identical to the formerly isolated strain DSM 2803, Bacillus thuringiensis subsp. tenebrionis (Krieg et al.: J.Appl.Ent. 104, 417-424, 1987). Furthermore, the nucleotide sequences and deduced amino acid sequences of the coleopteran active delta endotoxin genes from Bacillus thuringiensis subsp. tenebrionis and "Bacillus thuringiensis subsp. san diego" are identical.

Under the same culture conditions the above-mentioned second type of crystals are also synthesized by "Bacillus thuringiensis subsp. san diego" (A.M. Huger & A. Krieg, J.Appl.Ent. 108, 490-497, 1989).

According to H. de Barjac & E. Frachon (Entomophaga 35(2), 233-240, 1990) the "san diego" isolate is similar to "tenebrionis" and it cannot be justified to regard it as a different subspecies.

The utility of Bacillus thuringiensis strains for the control of coleopteran pests is dependent upon efficient and economical production of the coleopteran active toxins and the potency of the product produced. This in turn is dependent upon the amount of delta endotoxins which can be produced by fermentation of the coleopteran active Bacillus thuringiensis strains.

B. thuringiensis has been used for many years for the production of insecticides, but although mutants of B. thuringiensis with increased delta-endotoxins yield would be advantageous, no such mutants have previously been described. Mutants producing higher yields of delta-endotoxins would give a more efficient and economical production of B. thuringiensis toxins and a possibility for manufacture of B. thuringiensis products with increased potency at equal cost. This in turn would be an advantage for the user as reduced volumes of pesticide formulation have to be stored and handled for a given acreage. In addition, the users will have less container material to dispose of, thereby reducing the impact on the environment

Improvements of the production of delta endotoxin by Bacillus thuringiensis subsp. tenebrionis through mutation have not previously been reported.

One problem associated with the use of especially B. thuringiensis subspecies tenebrionis in controlling beetle larvae has been the relatively low potency or strength of the preparations requiring the application of relatively large amounts of preparation to the areas to be treated, such as 5 to 10 liter/ha compared to 1 to 2 liter/ha of most other B. thuringiensis products and most other insecticides.

Consequently a recognized need for products of improved strength exists.

One way to overcome this problem would be to concentrate the preparations. However, this would add considerably to the production cost in comparison to the savings obtained in storage and transportation.

A much more elegant solution would be to create mutants of existing B. thuringiensis strains capable of producing substantially larger amounts of delta endotoxins per cell.

### SUMMARY OF THE INVENTION

The present invention consequently in one aspect relates to variant or mutant Bacillus thuringiensis subsp. tenebrionis strains capable of producing substantially larger amounts of toxins than their parent strain.

Further aspects of the invention relate to the use of such variant or mutant Bacillus thuringiensis subsp. tenebrionis strains for the production of pesticidal products, and also to such pesticidal compositions comprising as the active ingredient delta-endotoxins produced by the variant or mutant Bacillus thuringiensis subsp. tenebrionis strains of the invention.

Also, the invention in one of its aspects relate to a method of controlling pests by applying a composition according to the invention to an area where pests susceptible to the activity of the delta-endotoxins in question are to be controlled.

In a still further aspect this invention relates to methods of selecting, or mutating and selecting B. thuringiensis subsp. tenebrionis strains in order to obtain such variated or mutated B. thuringiensis subsp. tenebrionis strains capable of producing substantially larger amounts of delta-endotoxins than their parent strain.

### DEPOSITION OF MICROORGANISMS

For the purpose of describing this invention in detail a mutant of Bacillus thuringiensis subsp. tenebrionis which produces high amounts of delta-endotoxin has been deposited with the Deutsche Sammlung von Mikroorganismen und Zellkulturen GmbH, Mascheroderwreg lb, D-3300 Braunschweig, Federal Republic of Germany, for the purposes of patent procedure on the date indicated below. DSM being an international depositary under the Budapest Treaty affords permanence of the deposit in accordance with rule 9 of said treaty.

| | |
|---|---|
| Deposit date | 10 August 1989 |
| Depositors ref. | NB 176-1 |
| DSM designation | DSM 5480 |

The mutant DSM 5480 was obtained by mutation, of Bacillus thuringiensis subsp. tenebrionis, strain DSM 5526, that has also been deposited under the Budapest Treaty as indicated below

| | |
|---|---|
| Deposit date | 14 September 1989 |
| Depositor's ref. | NB 125 |
| DSM designation | DSM 5526 |

### DETAILED DESCRIPTION OF THE INVENTION

This invention relates in its general aspect to variants or mutants of Bacillus thuringiensis subsp. tenebrionis producing high amounts of active delta-endotoxins as compared to its parent strain and having a parasporal crystal having a mean edge length of 2 µm or greater and a sporulation frequency at least 10 times lower than the sporulation frequency of the parent strain, and wherein the delta-endotoxins produced by said mutant or variant B. thuringienis subsp. tenebrionis is active against coleopterans. In this context the expression "high amounts" preferably means at least twice as much or more.

According to a preferred embodiment of the invention, the variated or mutated B. thuringiensis subsp. tenebrionis is capable of producing more than three times as much delta endotoxin as the strain DSM 2803.

A still further embodiment of the invention comprises varianted or mutated B. thuringiensis subsp. tenebrionis strains showing a sporulation frequency 10 to 100 or even 10⁶ times lower than the sporulation frequency of the parent strain or the strain DSM 2803.

An even more specific embodiment comprises the deposited mutant B. thuringiensis subsp. tenebrionis, DSM 5480.

While working on this invention a mutant of B. thuringiensis subsp. tenebrionis (DSM 5480) with more than a twofold increase in delta-endotoxin production as compared to its parent strain (DSM 5526) has been isolated. Phase contrast microscopy, scanning electron microscopy and transmission electron microscopy of this mutant indicate that the high productivity of this mutant is due to changes in the regulation of delta endotoxin production relative to sporulation resulting in the production of protein crystals which are up to more than five times bigger than the crystals produced by the existing coleopteran active Bacillus thuringiensis subsp. tenebrionis strains. The close correlation between crystal formation and sporulation seems to have been removed and the mutant produces high amounts of delta-endotoxin prior to sporulation.

In one of its aspects the invention relates to the use of the variated or mutated strains of the invention in a method for the production of insecticidal B. thuringiensis subsp. tenebrionis products, by which method the variant or mutant B. thuringiensis subsp. tenebrionis strain is cultivated in a suitable culture medium comprising sources for carbon, nitrogen, and other components known to the skilled person for a suitable period of time, whereafter the delta-endotoxins are recovered.

In a further aspect of the invention the B. thuringiensis subsp. tenebrionis delta-endotoxins product obtained as above is used in pesticidal compositions as an active component.

In such compositions the delta endotoxins of the invention may be utilized either alone or in combination with other biocidally active products.

The invention also relates to such pesticidal compositions or preparations comprising the B. thuringiensis subsp. tenebrionis delta-endotoxin product of the invention in admixture with agriculturally acceptable diluents or carriers.

The invention also relates to such pesticidal compositions or preparations comprising a B. thuringiensis subsp. tenebrionis delta-endotoxin product, which pesticidal composition in a liquid form has a potency of at least 15,000 BTTU/g, corresponding to at least 3% w/w coleopteran insecticidal crystal protein, or which pesticidal composition in a dry form has a potency of at least 50,000 BTTU/g, corresponding to at least 10% w/w coleopteran insecticidal crystal protein.

In a specific embodiment the invention relates to pesticidal compositions produced from DSM 5480 having at least twice the potency of pesticidal compositions produced from DSM 2803 or other coleopteran active Btt strains.

The compositions of the invention can take any form known in the art for the formulation of agrochemicals, for example, a suspension, a dispersion, an aqueous emulsion, a dusting powder, a dispersible powder, an emulsifiable concentrate or granules. Moreover it can be in a suitable form for direct application or as a concentrate or primary composition which requires dilution with a suitable quantity of water or other diluent before application.

The concentration of the insecticidally active B. thuringiensis subsp. tenebrionis delta-endotoxins in the compositions of the present invention when used alone or in combination with another pesticide, as applied to plants is preferably within the range from about 0.5 to about 25 per cent by weight, especially 1 to 15 per cent by weight.

In a still further aspect the invention relates to a method of controlling pests, wherein a pesticidal composition according to the preceding aspect is applied to an area infected with said pest.

In a specific embodiment, the coleopteran pest to be controlled is the colorado potato beetle.

In specific embodiments the pest can be controlled by the application of 23.4 ml/are (1 quart per acre) of a liquid pesticidal composition of the invention or by the application of 5.6 g/are (0.5 lb. per acre) of a dry pesticidal composition of the invention.

The active B. thuringiensis subsp. tenebrionis preparation or the compositions of the invention can be applied directly to the plant by, for example, spraying or dusting at the time when the pest has begun to appear on the plant. The preferred mode of application is by spraying. It is generally important to obtain good control of pests in the early stages of larval development as this is the time when the plant has suffered least damage.

In one method of mutating Bacillus thuringiensis subsp. tenebrionis strains and selecting such mutants that are capable of producing substantially larger amounts of delta-endotoxins than their parent strains, the parent strain is:
i) treated with a mutagen,
ii) the thus treated mutants are grown on a medium suitable for the selection of asporogenous and/or oligosporogenous strains,
iii) translucent colonies are selected and grown in a medium that does not fluidize on heating, and
iv) truly asporogenous strains are deselected by subjecting the colonies to a heat treatment.

According to a preferred embodiment of this method the thus selected colonies are grown in a normal production medium, and a final selection for strains capable of increasing the delta endotoxin production is performed.

In step (i) of the above method the mutagen may be any suitable conventional chemical mutagen, such as N-methyl-N'-nitro-N-nitrosoguanidine, or ethyl methanesulfonate, ,or the parent strain may be treated with electromagnetic radiation, such as γ-, X-ray-, or UV-radiation.

In step (ii) a suitable medium could be a modified nutrient sporulation medium including phosphate (NSMP medium) as described by Johnson et al., In "Spores VI": eds. P. Gerhardt et al., pp. 248-254, 1975.

In step (iv) of the method of the invention a suitable medium could be a NSMP medium supplemented with MgCl₂ and Gelrite, Kelco.

Another method of obtaining the high producing variants or mutants of the invention may be contemplated such as growing the parent strain in a liquid medium and selecting spontaneous mutants or variants after spreading the culture broth on an agar medium suitable for selection of asporogeneous and/or oligosporogenous mutants.

Other methods of screening for the high producing variants or mutants of the invention may be contemplated such as using the mass of these mutants directly through centrifugation or other means of separating for mass.

### Example 1

A mutant of B. thuringiensis subsp. tenebrionis with more than a twofold increase in delta-endotoxin production has been isolated. Phase contrast microscopy, scanning electron microscopy and transmission electron microscopy of this mutant indicate that the high productivity of this mutant is due to changes in the regulation of delta endotoxin production relative to sporulation resulting in the production of protein crystals which are up to more than five times bigger than the crystals produced by the existing coleopteran active Bacillus thuringiensis strains. The close correlation between crystal formation and sporulation seems to have been removed and the mutant produces high amounts of delta-endotoxin prior to sporulation.

### Production of High Yield Mutant

Spores of B. thuringiensis subsp. tenebrionis, strain DSM5526 were γ-irradiated to give a dosis of 7 kGy. The irradiated spores were spread onto NSMP agar plates (modified nutrient sporulation medium including phosphate as described by Johnson et al., In "Spores VI": eds. P. Gerhardt et al., pp. 248-254, 1975). A medium suitable for selection of asporogenous and/or oligosporogenous mutants.

The NSMP-agar plates were incubated at 30°C for 2-3 days. Translucent colonies were picked out and transferred to NSMP gelrite plates (NSMP medium supplemented with MgCl₂ (0.57 g/l) and Gelrite, Kelco (20 g/l)).

The NSMP gelrite plates were incubated for one hour at 90°C and then further incubated for 1-2 days at 30°C.

Mutants that grew well on the NSMP gelrite plates were selected. In this way all asporogenous mutants were deselected as they fail to grow after the heat treatment.

The selected mutants were grown in shakeflasks containing a commercial medium. The amounts of delta-endotoxin produced were determined by immunological methods described below.

Only mutants producing significantly higher amounts of delta-endotoxin than the parent strain were selected.

The morphology of the selected mutants on solid medium and in liquid media were studied by phase contrast microscopy (x 2500) and by scanning and transmission electron microscopy. The number of spores and crystals were counted and the size of the protein crystals were determined.

Among the mutants obtained, one (DSM 5480) was selected for its outstanding ability to produce delta-endotoxin.

The amount of delta endotoxin produced by mutant DSM 5480 was compared with that of DSM 2803 the original isolate of Bacillus thuringiensis subsp. tenebrionis, Bacillus thuringiensis subsp. tenebrionis, strain DSM 5526 used for the production of NOVODOR®, Bacillus thuringiensis subsp. tenebrionis, strain NB178, isolated from Sandoz' Bacillus thuringiensis tenebrionis product TRIDENT® from 1989, strain NB 198, isolated from Sandoz' B. thuringiensis subs. tenebrionis product TRIDENT® from 1990, "Bacillus thuringiensis subsp. san diego", strain NRRL-B-15939, and strain NB 197 isolated from Mycogen's B. thuringiensis subs. san diego" product M-ONE® from 1990. As shown in Table I of Example 2 the yield improved mutant of the invention produces 2-3.5 times as much delta endotoxin as the coleopteran active strains of Bacillus thuringiensis available today.

### Example 2

In this example the delta endotoxin yield of Bacillus thuringiensis subspecies tenebrionis,mutant DSM 5480 was compared with the delta endotoxin yields of Bacillus thuringiensis subspecies tenebrionis strains DSM 2803 (the original isolate of Bacillus thuringiensis subsp. tenebrionis), DSM 5526 (production strain of Novo-Nordisk), and NB 178 and NB 198 (production strains of Sandoz), and Bacillus thuringiensis subsp. san diego, strain NRRL-B 15939 and NB 197 (production strains of Mycogen) in a commercial medium. Each of the strains was grown for 17 hours at 30°C on agar slants of the following composition expressed as gram per litre of distilled water

| | |
|---|---|
| Peptone, Difco | 5 g |
| Beef extract, Difco | 3 g |
| Agar, Difco | 20 g |
| pH | 7.0 |

5 ml of a suspension of cells from each strain were then transferred to 100 ml of production medium in 500 ml baffle-bottom Erlenmeyer flasks. The production medium consisted of the following components in the quantities indicated (expressed as grams per litre of tap water).

| | |
|---|---|
| Soy bean meal | 50 g |
| Hydrolyzed starch | 40 g |
| KH₂PO₄ | 1.77 g |
| K₂HPO₄ | 4.53 g |
| pH | 7.0 |

The inoculated flasks were incubated at 30°C with shaking (250 rpm). After 96 hours of incubation the culture broths were assayed for delta endotoxin yields by immunological methods.

The amounts of delta endotoxin produced by the individual strains were determined by rocket immuno electrophoresis (RIE) and a photometric immuno assay (PIA) using antibodies raised against purified protein crystals from Bacillus thuringiensis subsp. tenebrionis.

400 mg of each culture broth were weighed. 7 ml trisodium phosphate buffer (0.125 M, pH 12) was added to each sample. The suspensions were shaken for 1 hour in order to solubilize the delta endotoxin proteins.

The samples were then centrifuged at 3.500 rpm for 15 minutes and the supernatants were tested for delta-endotoxin by rocket immuno electrophoresis against antiserum raised against purified protein crystals from B. thuringiensis subsp. tenebrionis. The amounts of delta endotoxin were determined relatively to a standard with known content of crystal protein.

The concentration of crystal protein was also determined by a photometric immuno assay. The crystal proteins were dissolved in an alkaline solution. The dissolved proteins were precipitated by their antibodies. The rate of this reaction was determined turbidimetrically. The amounts of delta-endotoxin were determined relatively to a standard with known content of crystal protein.

Crystal antigens for production of the antibodies used in the assays were obtained from crystals isolated from B. thuringiensis subsp. tenebrionis.

Polyclonal antibodies were raised by injecting rabbits subcutaneously every fortnight with 0.25 mg of crystal antigen.

The results obtained are shown in the following Tables Ia and Ib. Delta endotoxin yields are expressed as BTTU/g (units per g culture broth, determined by rocket immuno electrophoresis, RIE, or by a photometric immuno assay, PIA). The value used for pure B. thuringiensis subsp. tenebrionis crystal protein is 500,000 BTTU/g. The values indicated in table Ia below being averages of 6-7 independent fermentations, and those in Table Ib being averages of 3 independent fermentations.

**Table Ia**

| Delta endotoxin production by strains of Bacillus thuringiensis subsp. tenebrionis in shakeflasks | | |
|---|---|---|
| | Delta endotoxin yield | |
| Strain | RIE BTTU/g | PIA BTTU/g |
| DSM 2803 | 676 | 1293 |
| NRRL-B 15939 | 747 | 1126 |
| NB 178 | 986 | 1728 |
| DSM 5526 | 1097 | 1860 |
| DSM 5480 | 2382 | 4169 |

**Table Ib**

| Delta endotoxin production by strains of Bacillus thuringiensis subsp. tenebrionis in shakeflasks | |
|---|---|
| | Delta endotoxin yield |
| Strain | RIE BTTU/g |
| NB 197 | 1103 |
| NB 198 | 1237 |
| DSM 5480 | 2867 |

From Tables Ia and Ib it appears that DSM 5480 produces more than three times as much delta endotoxin as the original strain of Bacillus thuringiensis subsp. tenebrionis, DSM 2803 and "Bacillus thuringiensis subsp. san diego", strain NRRL-B15939 and more than twice the amount of delta endotoxin as the strains used today for the manufacture of commercial products of Bacillus thuringiensis subsp. tenebrionis.

Phase contrast microscopy, scanning electron microscopy and transmission electron microscopy of Bacillus thuringiensis subsp. tenebrionis, mutant DSM 5480 have revealed that the protein crystals produced by this mutant are much bigger than the corresponding protein crystals produced by Bacillus thuringiensis subsp. tenebrionis, strains DSM 2803, DSM 5526, NB178 and NB 198, and "Bacillus thuringiensis subsp. san diego", strains NRRL-B 15939 and NB 197.

Culture broth of Bacillus thuringiensis subsp. tenebrionis, mutant DSM 5480 was tested for activity against colerado potato beetle larvae. The increased amount of delta-endotoxin produced by mutant DSM 5480 as determined by the immunological methods was reflected in the biological activity against colerado potato beetle larvae.

### Example 3

In this example sporulation and parasporal crystal formation in B. thuringiensis subsp. tenebrionis, strains, DSM 2803, DSM 5526, NB 178 and NB 198, and mutant DSM 5480, and "B. thuringiensis subsp. san diego", strains NRRL-B 15939 and NB 197 were compared on solid medium and in liquid medium.

Each of the strains was grown for 2 days at 30°C on agar plates of the following composition expressed as gram per liter of distilled water

| | |
|---|---|
| Peptone, Difco | 5 g |
| Beef extract, Difco | 3 g |
| Agar, Difco | 20 g |
| pH | 7.0 |

Each of the strains was also grown in liquid medium. All strains were grown for 17 hours at 30°C on agar slants. 5 ml of a suspension of cells from each strain were then transferred to 500 ml baffle bottom Erlenmeyer flasks each containing 100 ml of medium.

The medium consisted of the following components in the quantities indicated (expressed as grams per liter of tap-water).
Liquid medium:

| | |
|---|---|
| Yeast extract | 5 g |
| Tryptone | 5 g |
| Glucose | 1 g |
| KH₂PO₄ | 0.8 g |
| pH | 7.0 |

The inoculated flasks were incubated at 30°C with shaking (250 rpm) for 96 hours.

The morphology of the strains on the solid medium and in the liquid medium was studied daily by phase contrast microscopy (x 2500). The number of spores and crystals were counted and the size of the parasporal crystals were determined. A few selected samples were also studied by scanning and transmission electron microscopy.

B. thuringiensis subsp. tenebrionis, strains DSM 2803, DSM 5526, NB 178 and NB 198, and "B. thuringiensis subsp. san diego", strains NRRL-B 15939 and NB 197 all sporulated well on both media. Before cell lysis each cell contained a spore and a parasporal crystal. The size of the crystals was from 0.4 to 0.9-1.1 µm in length by the time of cell lysis. The average size of the protein crystals being 0.6-0.7 µm in length.

Mutant DSM 5480 produced only few spores (<10⁶ spores/ml) on the solid medium and in the defined liquid medium. Before cell lysis most cells contained a huge protein crystal but no spore. The size of the protein crystals was from 0.4-0.7 µm to 5.0 µm, the average size of the protein crystals being 2.2-2.3 µm in length.

Ultrastructural analysis of cells from these media by transmission electron microscopy revealed that the sporulation process in the mutant had been started but had not been completed by the time of cell lysis. The sporulation process had reached different stages in the various cells. In cells where the sporulation had only reached stage II (forespore septum formation) the protein crystals filled up the entire cells.

In the production medium (Example 2) the mutant produced a higher number of spores (10⁷-10⁸ spores/ml). In this medium the sporulation frequency of the mutant was 10-100 times lower than in the parent strain.

Thus the mutant has retained its ability to produce normal spores. However, the sporulation frequency of the mutant seems to be strongly dependent of the media.

The size of the protein crystals produced by the individual strains are shown in Tables IIa and IIb.

**Table IIa**

| Size of the protein crystals produced by coleopteran active B. thuringiensis strains available today. | | | |
|---|---|---|---|
| | Length of protein crystals in µm | | |
| Strain | Minimum value | Maximum value | Mean value |
| DSM 2803 | 0.4 | 0.9 | 0.7 |
| NRRL-B-15939 | 0.4 | 0.9 | 0.7 |
| NB 178 | 0.5 | 0.9 | 0.7 |
| DSM 5526 | 0.4 | 0.9 | 0.7 |
| DSM 5480 | 0.7 | 5.0 | 2.3 |

**Table IIb**

| Size of the protein crystals produced by coleopteran active B. thuringiensis strains available today. | | | |
|---|---|---|---|
| | Length of protein crystals in µm | | |
| Strain | Minimum value | Maximum value | Mean value |
| NB 197 | 0.4 | 0.7 | 0.6 |
| NB 198 | 0.4 | 1.1 | 0.7 |
| DSM 5480 | 0.4 | 4.2 | 2.0 |

From Tables IIa and IIb it is clear that mutant DSM 5480 produces much bigger protein crystals than any of the coleopteran active B.t. strains available today.

From the data obtained it appears that the regulation of delta endotoxin production in relation to sporulation has been changed in the mutant.

The mutant seems to produce the protein crystals prior to the development of spores hereby giving the cells a longer period for delta endotoxin production which result in the production of much bigger protein crystals by the time of cell lysis than in the parent strain.

Depending on the available nutrients and the size of the protein crystals in the cells by the time of sporulation a normal spore will be developed before the time of cell lysis.

### Example 4

In this example the high yielding Btt mutant DSM 5480 was used to produce high potency products for the control of colorado potato beetle larvae.

DSM 5480 was fermented on the production fermentation medium described in example 2 in an aerated, stirred production fermentation tank. After 96 hours, the broth was recovered by centrifugation on a continuous centrifuge.

The concentrated cream which contains the active protein crystals was stabilized by addition of microbial preservatives and pH was adjusted to 5.0.

One portion of the concentrated cream was spray dried and later used for the formulation of wettable powder. The rest of the concentrated cream was used directly for formulation of two aqueous flowable concentrates (FC).

The wettable powder was formulated as described in Table III. The formulation of the two FC's is described in Table IV.

**Table III**

| NOVODOR® wettable powder formulation | |
|---|---|
| Component | % by weight |
| Spray dried concentrated cream of Btt | 40 |
| Detergents | 9 |
| Anticaking agent | 1 |
| Inert filler | 50 |

**Table IV**

| NOVODOR® FC formulations | | |
|---|---|---|
| Component | NOVODOR® FC 1 % by weight | NOVODOR® FC 2 % by weight |
| Btt concentrated cream | 80 | 55 |
| Preservatives | 4 | 4 |
| Antifreeze agents | 9.1 | 19 |
| Detergents | 2.5 | 2.5 |
| pH regulator | 2.85 | 2.85 |
| water | 1.55 | 16.65 |
| | 100.0 | 100.0 |

When using a value of 500,000 BTTU/g of pure crystal protein the content of active crystal protein in the formulations are:

| | | % Btt crystal protein |
|---|---|---|
| NOVODOR® WP | 70.8 KBTTU/g | 14.16 |
| NOVODOR® FC1 | 24.7 KBTTU/g | 4.94 |
| NOVODOR® FC2 | 14.2 KBTTU/g | 2.84 |

The detergents were chosen among the wide selection of suspension aids and wetting agents normally used in agricultural pesticide products.

The anticaking agent is a hydrophilic silica and the inert filler was chosen from the generally used inert fillers such as bentonites, inorganic salts or clays.

The preservates used in the FC's were chosen from the group of food and cosmetic preservatives. The pH regulator is an inorganic acid.

### Example 5

A field trial was conducted to prove the biological effect of the high yielding Btt mutant DSM 5480 on the main target pest, colorado potato beetle larvae. In comparison with the two commercial products Trident® and M-one®. The crop was potatoes.

The crop was sprayed 3 times on July 20th, July 27th and August 3rd (2nd generation larvae).
The products and dosages used were:

| | Product volume/acre* | Potency KBTTU/g | % Btt crystal protein in the formulation |
|---|---|---|---|
| NOVODOR® FC 2 | 1 qt/acre | 14.2 | 2.84 |
| | 1.5 qts/acre | 14.2 | 2.84 |
| | 2.5 qts/acre | 14.2 | 2.84 |
| | 3.0 qts/acre | 14.2 | 2.84 |
| TRIDENT® | 4 qts/acre | 5.5 | 1.10 |
| M-one® | 2 qts/acre | 8.9 | 1.78 |

| | | | |
|---|---|---|---|
| *1qt/acre = 23.4 ml/are | | | |

The mean % control of CPB larvae compared to the untreated control is given in table V. The colorado potato beetle pressure was very heavy in the untreated control: 370 larvae per 20 plants on August 1st and 904 larvae per 20 plants on August 8th.

**Table V**

| | % control | |
|---|---|---|
| Treatment | August 1st | August 8th |
| NOVODOR® FC 2 1 qt** | 99 | 99 |
| NOVODOR® FC 2 1.5 qts | 95 | 100 |
| NOVODOR® FC 2 2.5 qts | 98 | 99 |
| NOVODOR® FC-2 3 qts | 100 | 100 |
| TRIDENT® 4 qts | 94 | 98 |
| M-one® 2 qts | 98 | 98 |

| | | |
|---|---|---|
| ** 1 qt = 0.95 liter | | |

These results clearly show that products made with the high yielding mutant DSM 5480 are effective for the control of colorado potato beetle larvae in the field. The crystal protein produced by the high yielding strain is fully active as 1.4 liter (1.5 qts) NOVODOR® FC give as good results as Trident at 3.8 liter (4 qts) and as good as M-one at 1.9 liter (2 qts).

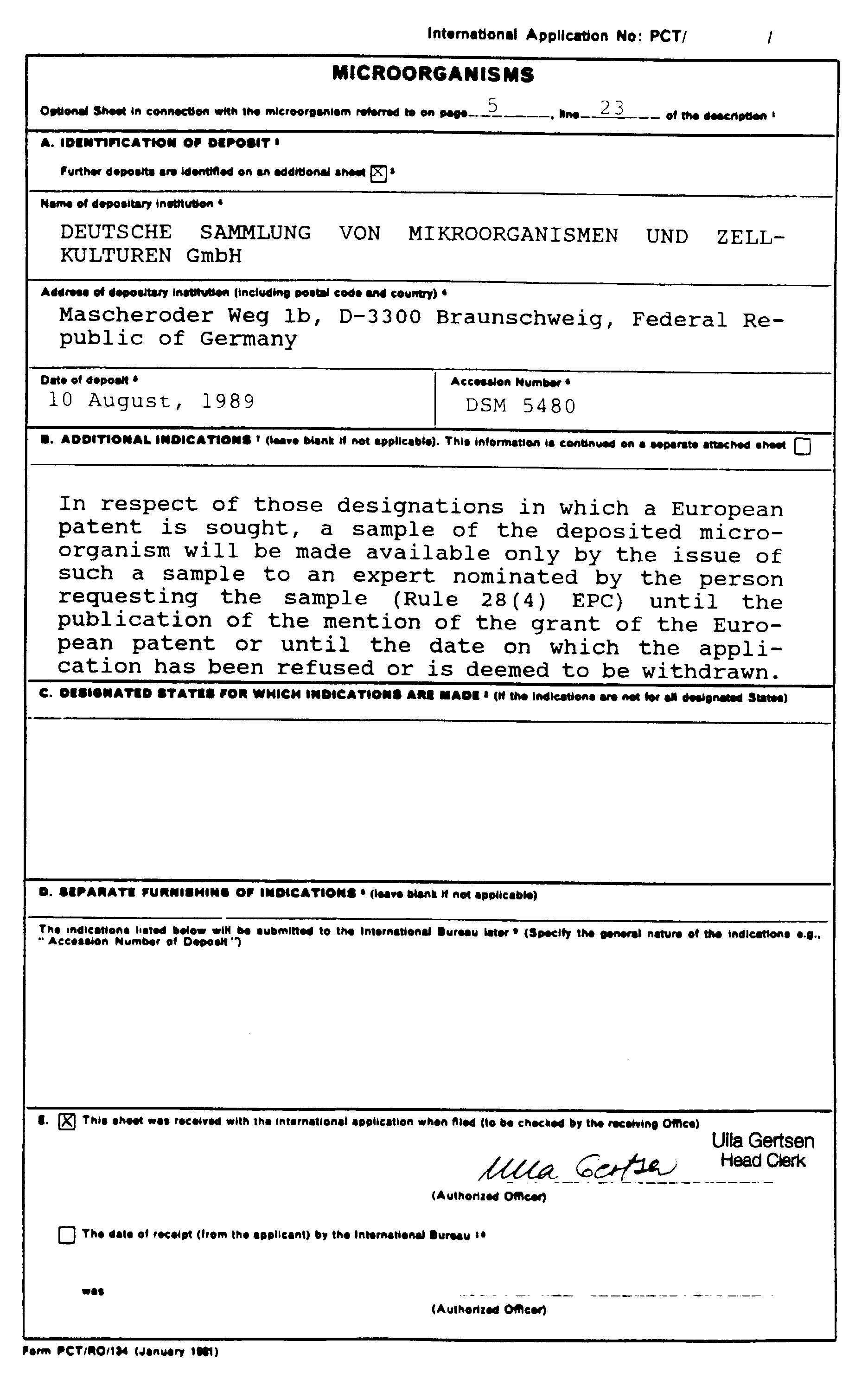
International Application No: PCT/ /
MICROORGANISMS
Optional Sheet in connection with the microorganism referred to on page 5 , line 23 of the description ¹
A. IDENTIFICATION OF DEPOSIT ³
Further deposits are identified on an additional sheet [X] ³
Name of depositary institution ⁴
DEUTSCHE SAMMLUNG VON MIKROORGANISMEN UND ZELL-
KULTUREN GmbH
Address of depositary institution (including postal code and country) ⁴
Mascheroder Weg 1b, D-3300 Braunschweig, Federal Re-
public of Germany
Date of deposit ⁵ | Accession Number ⁶
10 August, 1989 | DSM 5480
B. ADDITIONAL INDICATIONS ⁷ (leave blank if not applicable). This information is continued on a separate attached sheet [ ]
In respect of those designations in which a European
patent is sought, a sample of the deposited micro-
organism will be made available only by the issue of
such a sample to an expert nominated by the person
requesting the sample (Rule 28(4) EPC) until the
publication of the mention of the grant of the Euro-
pean patent or until the date on which the appli-
cation has been refused or is deemed to be withdrawn.
C. DESIGNATED STATES FOR WHICH INDICATIONS ARE MADE ⁸ (if the indications are not for all designated States)
D. SEPARATE FURNISHING OF INDICATIONS ⁸ (leave blank if not applicable)
The indications listed below will be submitted to the International Bureau later ⁹ (Specify the general nature of the indications e.g.,
" Accession Number of Deposit ")
E. [X] This sheet was received with the international application when filed (to be checked by the receiving Office)
Ulla Gertsen
Head Clerk
(Authorized Officer)
[ ] The date of receipt (from the applicant) by the International Bureau ¹⁰
was
(Authorized Officer)
Form PCT/RO/134 (January 1981)

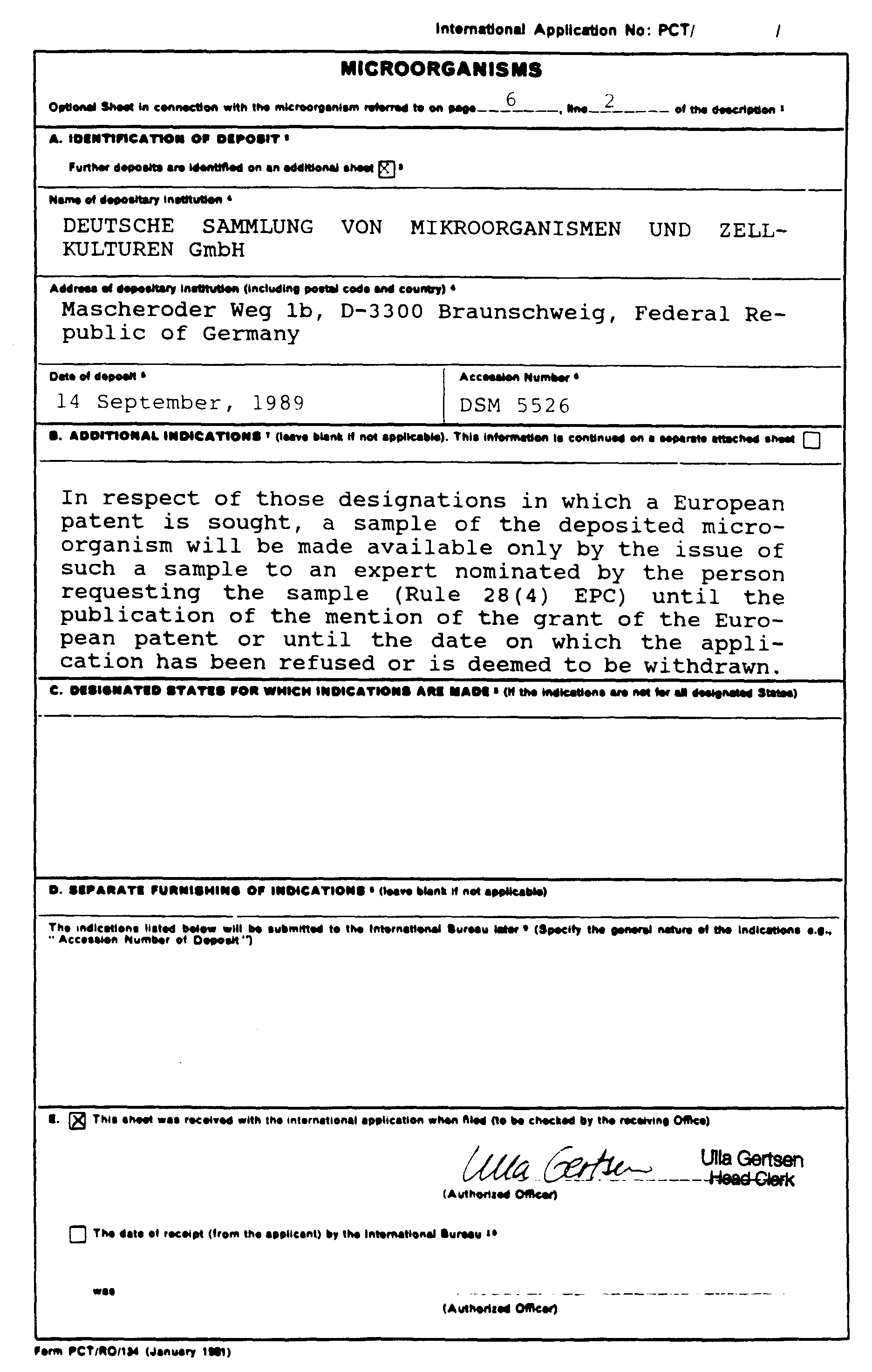
International Application No: PCT/ /
MICROORGANISMS
Optional Sheet in connection with the microorganism referred to on page 6 , line 2 of the description ¹
A. IDENTIFICATION OF DEPOSIT ³
Further deposits are identified on an additional sheet [X] ³
Name of depositary institution ⁴
DEUTSCHE SAMMLUNG VON MIKROORGANISMEN UND ZELL-
KULTUREN GmbH
Address of depositary institution (including postal code and country) ⁴
Mascheroder Weg 1b, D-3300 Braunschweig, Federal Re-
public of Germany
Date of deposit ⁵ | Accession Number ⁶
14 September, 1989 | DSM 5526
B. ADDITIONAL INDICATIONS ⁷ (leave blank if not applicable). This information is continued on a separate attached sheet [ ]
In respect of those designations in which a European
patent is sought, a sample of the deposited micro-
organism will be made available only by the issue of
such a sample to an expert nominated by the person
requesting the sample (Rule 28(4) EPC) until the
publication of the mention of the grant of the Euro-
pean patent or until the date on which the appli-
cation has been refused or is deemed to be withdrawn.
C. DESIGNATED STATES FOR WHICH INDICATIONS ARE MADE ⁸ (If the indications are not for all designated States)
D. SEPARATE FURNISHING OF INDICATIONS ⁸ (leave blank if not applicable)
The indications listed below will be submitted to the International Bureau later ⁹ (Specify the general nature of the indications e.g.,
" Accession Number of Deposit ")
E. [X] This sheet was received with the international application when filed (to be checked by the receiving Office)
 Ulla Gertsen
Head Clerk
(Authorized Officer)
[ ] The date of receipt (from the applicant) by the International Bureau ¹⁰
was
(Authorized Officer)
Form PCT/RO/134 (January 1981)

## Claims (Claims for the following Contracting State(s): AT, BE, CH, LI, DE, DK, FR, GB, GR, IT, LU, NL, SE)

1. A mutant or variant of a Bacillus thuringiensis subsp. tenebrionis strain capable of producing high amounts of insecticidal delta-endotoxins as compared to its parent strain and having a parasporal crystal having a mean edge length of 2 µm or greater and a sporulation frequency at least 10 times lower than the sporulation frequency of the parent strain, and wherein the delta-endotoxins produced by said mutant or variant B. thuringiensis subsp. tenebrionis are active against coleopterans.

2. The mutant or variant according to claim 1, wherein the mutant or variant B. thuringiensis subsp. tenebrionis strain is capable of producing twice as much or more of insecticidal delta-endotoxins as compared to its parent strain.

3. The mutant or variant according to claim 1 or 2, wherein the mutant or variant B. thuringiensis subsp. tenebrionis strain belongs to the pathotype C of B. thuringiensis subsp. tenebrionis.

4. The mutant or variant according to claim 1, 2 or 3 wherein the mutant or variant B. thuringiensis subsp. tenebrionis strain is capable of producing more than three times as much delta-endotoxin as the strain DSM 2803.

5. The mutant or variant according to claim 3 or 4, wherein the mutant or variant B. thuringiensis subsp. tenebrionis strain shows a sporulation frequency 10 to 100 or even 10⁶ times lower than the sporulation frequency of the strain DSM 2803.

6. The mutant according to claim 1, wherein the mutant is the deposited mutant B. thuringiensis subsp. tenebrionis strain DSM 5480.

7. A process for producing a B. thuringiensis subsp. tenebrionis delta-endotoxin product by use of a mutant or variant strain according to any of the claims 1 to 6, whereby the mutant or variant B. thuringiensis strain is cultivated in a suitable culture medium comprising sources for carbon, nitrogen, and other components for a suitable period of time, whereafter an insecticidal product comprising the delta-endotoxins are recovered alone or in combination with cells and/or spores from the culture medium, and this product optionally is admixed with agriculturally acceptable excipients.

8. A pesticidal composition comprising the B. thuringiensis subsp. tenebrionis delta-endotoxin product obtainable in accordance with claim 7 as an active component.

9. The pesticidal composition according to claim 8, wherein the delta-endotoxin product is utilized either alone or in combination with other biocidally active products.

10. The pesticidal composition or preparation according to claim 8 or 9, comprising the B. thuringiensis subsp. tenebrionis delta-endotoxin product in admixture with an agriculturally acceptable diluent or carrier.

11. The pesticidal composition according to claim 8, 9 or 10 having a potency more than twice that of pesticidal compositions produced with the parent strain.

12. A liquid pesticidal composition according to claim 10 having a potency of at least 15,000 BTTU/g, corresponding to at least 3% w/w coleopteran insecticidal crystal protein.

13. A dry pesticidal composition according to claim 10 having a potency of at least 50,000 BTTU/g, corresponding to at least 10% w/w coleopteran insecticidal crystal protein.

14. A pesticidal composition produced from DSM 5480 having at least twice the potency of pesticidal compositions produced from DSM 2803 or other coleopteran active Btt strains.

15. A composition according to any of claims 8-14, wherein the diluent or carrier in the composition is a solid or a liquid optionally in association with a surface-active agent.

16. A method of controlling pests, wherein a pesticidal composition according to any of the claims 8 to 15 is applied to an area infected with said pest.

17. The method of claim 16, wherein the pest is a coleopteran, especially colorado potato beetle.

18. The method of claim 17, wherein said pesticidal composition is a liquid pesticidal composition and wherein the pest can be controlled by the application of 23.4 ml/are (1 quart per acre) of said liquid pesticidal composition.

19. The method of claim 17, wherein said pesticidal composition is a dry pesticidal composition and wherein the pest can be controlled by the application of 5.6 gram/are (0.5 lb. per acre) of said dry pesticidal composition.

20. A method of mutating Bacillus thuringiensis subsp. tenebrionis strains and selecting such mutants according to claim 1 that are capable of producing substantially larger amounts of delta-endotoxins than their parent strains, wherein the parent strain is:
i) treated with a mutagen,
ii) the thus treated mutants are grown in a medium suitable for the selection of asporogenous and/or oligosporogenous strains,
iii) translucent colonies are selected and grown in a medium that does not fluidize on heating, and
iv) truly asporogenous strains are deselected by subjecting the colonies to a heat treatment.

21. The method of claim 20, wherein the thus selected colonies are grown in a normal production medium, and a final selection for strains capable of increasing the delta-endotoxin production is performed.

22. The method of claim 20 or 21, wherein in step (i) the mutagen is any suitable chemical mutagen, such as N-methyl-N'-nitro-N-nitrosoguanidine, or ethyl methanesulfonate.

23. The method of claim 20 or 21, wherein the parent strain in step (i) is treated with a suitable eletromagnetic radiation, such as γ-, or X-ray-, or UV-radiation.

24. The method of any one of claims 20 to 23, wherein in step (ii) said medium is a modified nutrient sporulation medium including phosphate (NSMP medium).

25. The method of any one of the claims 20 to 24, wherein in step (iv) said medium is a NSMP medium supplemented with MgCl₂ and Gelrite, Kelco.

## Claims (Claims for the following Contracting State(s): ES)

1. A process for producing a Bacillus thuringiensis subsp. tenebrionis delta-endotoxin product by use of a mutant or variant of a B. thuringiensis subsp. tenebrionis strain capable of producing high amounts of insecticidal delta-endotoxins as compared to its parent strain and having a parasporal crystal having a mean edge length of 2 µm or greater and a sporulation frequency at least 10 times lower than the sporulation frequency of the parent strain, and wherein the delta-endotoxins produced by said mutant or variant B. thuringiensis subsp. tenebrionis are active against coleopterans, said process comprising cultivating said mutant or variant B. thuringiensis subsp. tenebrionis strain in a suitable culture medium comprising sources for carbon, nitrogen, and other components for a suitable period of time, whereafter an insecticidal product comprising the delta-endotoxins are recovered alone or in combination with cells and/or spores from the culture medium, and this product is optionally formulated to prepare a pesticidal composition.

2. A process according to claim 1, wherein the mutant or variant strain of Bacillus thuringiensis subsp. tenebrionis is obtained by mutation and selection of Bacillus thuringiensis subsp. tenebrionis strains, wherein the parent strain is:
(i) treated with a mutagen,
(ii) the thus treated mutants are grown in a medium suitable for the selection of asporogenous and/or oligosporogenous strains,
(iii) translucent colonies are selected and grown in a medium that does not fluidize on heating, and
(iv) truly asporogenous strains are deselected by subjecting the colonies to a heat treatment.

3. The process according to claim 1 or 2, wherein the mutant or variant B. thuringiensissubsp. tenebrionis strain is capable of producing twice as much or more of insecticidal delta-endotoxins as compared to its parent strain.

4. The process according to claim 1, 2 or 3, wherein the mutant or variant B. thuringiensis subsp. tenebrionis strain belongs to the pathotype C of B. thuringiensis subsp. tenebrionis.

5. The process according to claim 1, 2, 3 or 4, wherein the mutant or variant B. thuringiensis subsp. tenebrionis strain is capable of producing more than three times as much delta-endotoxin as the strain DSM 2803.

6. The process according to claim 4 or 5, wherein the mutant or variant B. thuringiensis subsp. tenebrionis strain shows a sporulation frequency 10 to 100 or even 10⁶ times lower than the sporulation frequency of the strain DSM 2803.

7. The process according to claim 1 or 2, wherein the mutant is the deposited mutant B. thuringiensis subsp. tenebrionis strain DSM 5480.

8. The process according to claim 2, wherein the thus selected colonies are grown in a normal production medium, and a final selection for strains capable of increasing the delta-endotoxin production is performed.

9. The process according to claim 2 or 8, wherein in step (i) the mutagen is any suitable chemical mutagen, such as N-methyl-N'-nitro-N-nitrosoguanidine, or ethyl methanesulfonate.

10. The process according to claim 2 or 8, wherein the parent strain in step (i) is treated with a suitable electromagnetic radiation, such as γ-, or X-ray, or UV-radiation.

11. The process according to any one of claims 2 and 8-10, wherein in step (ii) said medium is a modified nutrient sporulation medium including phosphate (NSMP medium).

12. The process according to any one of claims 2 and 8-11, wherein in step (iv) said medium is a NSMP medium supplemented with MgCl₂ and Gelrite, Kelco.

13. The process according to claim 1, wherein the Bacillus thuringiensis subsp. tenebrionis delta-endotoxin product is formulated with agriculturally acceptable excipients to obtain a pesticidal composition wherein said product constitutes the active ingredient.

14. The process according to claim 13, wherein said delta-endotoxin product is formulated either alone or in combination with other biocidally active products to produce the pesticidal composition.

15. The process according to claim 13 or 14, wherein the delta-endotoxin product is combined with an agriculturally acceptable diluent or carrier to obtain the pesticidal composition.

16. The process according to claim 13, 14 or 15, wherein the pesticidal composition has a potency more than twice that of pesticidal compositions produced with the parent strain.

17. The process according to claim 16, wherein the pesticidal composition has a potency of at least 15,000 BTTTU/g, corresponding to at least 3% w/w coleopteran insecticidal crystal protein.

18. The process according to claim 16, wherein the pesticidal composition has a potency of at least 50,000 BTTTU/g, corresponding to at least 10% w/w coleopteran insecticidal crystal protein.

19. The process according to claim 7, wherein the pesticidal composition has at least twice the potency of pesticidal compositions produced from DSM 2803 or other coleopteran active Btt strains.

20. The process according to any one of claims 13 to 19, wherein the diluent or carrier in the composition is a solid or a liquid optionally in association with a surface-active agent.

21. Use of the B. thuringiensis subsp. tenebrionis delta-endotoxin product, obtainable in acordance with the process of any one of claims 1 and claims 3-7 dependent therefrom, in the production of pesticidal compositions that contain it as an active component, for application to an area infected with said pest.

22. The use according to claim 21, wherein the delta-endotoxin product is utilized either alone or in combination with other biocidally active products.

23. The use according to claim 21 or 22, wherein the delta-endotoxin product is utilized in admixture with an agriculturally acceptable diluent or carrier.

24. The use according to claim 21, 22 or 23, wherein the pesticidal composition has a potency more than twice that of pesticidal compositions produced with the parent strain.

25. The use according to claim 23, wherein the pesticidal composition is a liquid pesticidal composition having a potency of at least 15,000 BTTU/g, corresponding to at least 3% w/w coleopteran insecticidal crystal protein.

26. The use according to claim 23, wherein the pesticidal composition is a dry pesticidal composition having a potency of at least 50,000 BTTU/g, corresponding to at least 10% w/w coleopteran insecticidal crystal protein.

27. The use according to claim 21, wherein the delta-endotoxin product is produced from the DSM 5480 strain.

28. The use according to any one of claims 21-27, wherein the diluent or carrier in the composition is a solid or a liquid optionally in association with a surface-active agent.

29. The use according to any one of claims 21-28, wherein the pest is a coleopteran, especially colorado potato beetle.

30. The use according to claim 29, wherein said pesticidal composition is a liquid pesticidal composition and wherein said pest can be controlled by the application of 23.4 ml/are (1 quart per acre) of said liquid pesticidal composition.

31. The use according to claim 29, wherein said pesticidal composition is a dry pesticidal composition and wherein said pest can be controlled by the application of 5.6 gram/are (0.5 lb. per acre) of said dry pesticidal composition.

32. Pesticidal composition, characterized in that it comprises
(i) 0.5 to 25% w/w of a delta-endotoxin product of a B. thuringiensis subsp. tenebrionis mutant or variant, obtained by the process of claim 1; and
(ii) an agriculturally acceptable excipient.

33. The pesticidal composition according to claim 32, wherein the delta-endotoxin product is utilized either alone or in combination with other biocidally active products.

34. The pesticidal composition according to claim 32 or 33, wherein the delta-endotoxin product is utilized in admixture with an agriculturally acceptable diluent or carrier.

35. The pesticidal composition according to claim 32, 33 or 34 having a potency more than twice that of pesticidal compositions produced with the parent strain.

36. A liquid pesticidal composition according to claim 34 having a potency of at least 15,000 BTTU/g, corresponding to at least 3% w/w coleopteran insecticidal crystal protein.

37. A dry pesticidal composition according to claim 34 having a potency of at least 50,000 BTTU/g, corresponding to at least 10% w/w coleopteran insecticidal crystal protein.

38. A pesticidal composition according to claim 32 wherein the delta-endotoxin product is produced from DSM 5480 having at least twice the potency of pesticidal compositions produced from DSM 2803 or other coleopteran active Btt strains.

39. A composition according to any one of claims 32-37 wherein the diluent or carrier in the composition is a solid or a liquid optionally in association with a surface-active agent.

## Patentansprüche (Patentansprüche für folgende(n) Vertragsstaat(en): AT, BE, CH, LI, DE, DK, FR, GB, GR, IT, LU, NL, SE)

1. Eine Mutante oder Variante eines Bacillus thuringiensis subsp. tenebrionis Stammes, die große Mengen an insektiziden delta-Endotoxinen, verglichen mit ihrem Urstamm herstellen kann und die einen parasporalen Kristall mit einer mittleren Kantenlänge von 2µm oder mehr aufweist und eine Sporulationsfrequenz, die wenigstens 10 mal niedriger ist, als die Sporulationsfrequenz des Urstammens, und wobei die delta-Endotoxine, die von dieser Mutante oder Varinate von B. thuringiensis subsp. tenebrionis produziert werden, gegen Coleoptera aktiv sind.

2. Mutante oder Variante nach Anspruch 1, wobei die Mutante oder Variante des B. thuringiensis subsp. tenebrionis Stammes in der Lage ist, doppelt soviel oder mehr insektizide delta-Endotoxine herzustellen, wie ihr Urstamm.

3. Mutante oder Variante nach Anspruch 1 oder 2, wobei die Mutante oder Variante des B. thuringiensis subsp. tenebrionis Stammes zum Pathotyp C von B. thuringiensis subsp. tenebrionis zählt.

4. Mutante oder Variante nach Anspruch 1, 2 oder 3, wobei die Mutante oder Variante des B. thuringiensis subsp. tenebrionis Stammes zur Herstellung von mehr als drei mal soviel delta-Endotoxin befähigt ist, wie der Stamm DSM 2803.

5. Mutante oder Variante nach Anspruch 3 oder 4, wobei die Mutante oder Variante des B. thuringiensis subsp. tenebrionis Stammes eine Sporulationsfrequenz aufweist, die 10 bis 100 oder sogar 10⁶ mal niedriger ist, als die Sporulationsfrequenz des Stammes DSM 2803.

6. Mutante nach Anspruch 1, wobei die Mutante die hinterlegte Mutante B. thuringiensis subsp. tenebrionis Stamm DSM 5480 ist.

7. Verfahren zur Herstellung eines B. thuringiensis subsp. tenebrionis delta-Endotoxin-Produktes unter Verwendung eines Mutanten oder varianten Stammens gemäß irgendeinem der Ansprüche 1 bis 6, wobei die Mutante oder Variante des B. thuringiensis Stammes in einem geeigneten Kulturmedium aufgezogen wird, das Quellen für Kohlenstoff, Stickstoff und andere Komponenten für einen geeigneten Zeitraum lang umfaßt, wonach ein insektizides Produkt, das die delta-Endotoxine umfaßt, alleine oder zusammen mit Zellen und/oder Sporen aus dem Kulturmedium erhalten wird, und wobei dieses Produkt wahlweise mit landwirtschaftlich verträglichen Hilfsstoffen vermengt wird.

8. Pestizide Zusammensetzung, die als aktiven Bestandteil das B. thuringiensis subsp. tenebrionis delta-Endotoxin-Produkt umfaßt, das in Übereinstimmung mit Anspruch 7 erhältlich ist.

9. Pestizide Zusammensetzung nach Anspruch 8, wobei das delta-Endotoxin-Produkt entweder alleine oder in Kombination mit anderen biozid aktiven Produkten verwendet wird.

10. Pestizide Zusammensetzung oder Präparat nach Anspruch 8 oder 9, das das B. thuringiensis subsp. tenebrionis delta-Endotoxin-Produkt in Vermengung in einem landwirtschaftlich verträglichen Verdünner oder Träger umfaßt.

11. Pestizide Zusammensetzung nach Anspruch 8, 9 oder 10, mit einer Wirksamkeit, die mehr als doppelt so stark wie diejenige der pestiziden Zusammensetzungen ist, die mit dem Urstamm hergestellt werden.

12. Flüssige pestizide Zusammensetzung nach Anpruch 10, die eine Wirksamkeit von wenigstens 15,000 BTTU/g aufweist, was wenigstens 3 Gewichtsprozent an gegenüber Coleoptera insektizidem Kristallprotein entspricht.

13. Trockene pestizide Zusammensetzung nach Anspruch 10, die eine Wirksamkeit von wenigstens 50,000 BTTU/g aufweist, was wenigstens 10 Gewichtsprozent an gegenüber Coleoptera insektizidem Kristallprotein entspricht.

14. Pestizide Zusammensetzung, die aus DSM 5480 hergestellt wird, die wenigstens die doppelte Wirksamkeit der pestiziden Zusammensetzungen aufweist, die aus DSM 2803 oder anderen gegenüber Coleoptera aktiven Btt Stämmen hergestellt werden.

15. Zusammensetzung nach irgendeinem der Ansprüche 8-14, wobei der Verdünner oder Träger in der Zusammensetzung ein Feststoff oder eine Flüssigkeit ist, wahlweise unter Miteinbeziehung eines oberflächenaktiven Mittels.

16. Verfahren zur Bekämpfung von Schädlingen, wobei eine pestizide Zusammensetzung nach irgendeinem der Ansprüche 8 bis 15 auf eine Fläche aufgebracht wird, die von dem Schädling befallen ist.

17. Verfahren nach Anspruch 16, wobei der Schädling zu den Coleoptera zählt, speziell der Coloradokartoffelkäfer.

18. Verfahren nach Anspruch 17, wobei die pestizide Zusammensetzung eine flüssige pestizide Zusammensetzung ist, und wobei der Schädling durch das Aufbringen von 23,4ml/are (1 quart per acre) der flüssigen pestiziden Zusammensetzung bekämpft werden kann.

19. Verfahren nach Anspruch 17, wobei die pestizide Zusammensetzung eine trockene pestizide Zusammensetzung ist, und wobei der Schädling durch das Aufbringen von 5,6gram/are (0,5lb per acre) der trockenen pestiziden Zusammensetzung bekämpft werden kann.

20. Verfahren zur Mutation von Bacillus thuringiensis subsp. tenebrionis Stämmen und zur Auswahl solcher Mutanten gemäß Anspruch 1, die zur Erzeugung von im wesentlichen größeren Mengen an delta-Endotoxinen als ihre Urstämme befähigt sind, wobei der Urstamm folgenden Schritten unterworfen wird:
i) Behandlung mit einem Mutagen,
ii) Aufzucht der so behandelten Mutanten in einem Medium, das für die Auswahl von asporogenen und/oder oligosporogenen Stämmen geeignet ist,
iii) Auswahl durchscheinender Kolonien und Aufzucht in einem Medium, das sich bei Erwärmung nicht verflüssigt und
iv) Deselektion von echt asporogenen Stämmen durch Hitzebehandlung der Kolonien.

21. Verfahren nach Anspruch 20, wobei die so ausgewählten Kolonien in einem Normalproduktionsmedium aufgezogen werden, und wobei eine Endselektion für Stämme, die zur Erhöhung der delta-Endotoxin-Produktion befähigt sind, durchgeführt wird.

22. Verfahren nach Anspruch 20 oder 21 wobei in Schritt (i) das Mutagen jedwedes geeignete chemische Mutagen ist, wie etwa N-Methyl-N'-nitro-N-nitrosoguanidin oder Ethylmethansulfonat.

23. Verfahren nach Anspruch 20 oder 21, wobei der Urstamm in Schritt (i) mit geeigneter elektromagnetischer Strahlung behandelt wird, wie etwa γ-, oder Röntgen- oder UV-Strahlung.

24. Verfahren nach irgendeinem der Ansprüche 20 bis 23, wobei in Schritt (ii) das Medium ein abgewandeltes Sporulationsnährmedium ist, das Phosphat enthält (NSMP Medium).

25. Verfahren nach irgendeinem der Ansprüche 20 bis 24, wobei in Schritt (iv) das Medium ein NSMP Medium ist, das mit MgCl₂ und Gelrite, Kelco bezusatzt ist.

## Patentansprüche (Patentansprüche für folgende(n) Vertragsstaat(en): ES)

1. Verfahren zur Herstellung eines Bacillus thuringiensis subsp. tenebrionis delta-Endotoxin-Produktes durch Verwendung einer Mutante oder Variante eines B. thuringiensis subsp. tenebrionis Stammes, der zu Herstellung von großen Mengen von insektiziden delta-Endotoxinen, verglichen mit seinem Urstamm, befähigt ist, und der einen parasporalen Kristall mit einer mittleren Kantenlänge von 2µm oder mehr aufweist, sowie eine Sporulationsfrequenz, die wenigstens 10 mal geringer ist, als die Sporulationsfrequenz des Urstammes, und wobei die delta-Endotoxine, die von der Mutante oder Variante von B. thuringiensis subsp. tenebrionis hergestellt werden, gegen Coleoptera aktiv sind, wobei das Verfahren die Aufzucht der Mutante oder Variante des B. thuringiensis subsp. tenebrionis Stammes in einem geeigneten Kulturmedium umfaßt, das Quellen für Kohlenstoff, Stickstoff und andere Bestandteile für einen geeigneten Zeitraum lang enthält, wonach das insektizide Produkt, das die delta-Endotoxine umfaßt, allein oder in Kombination mit den Zellen und/oder Sporen aus dem Kulturmedium isoliert wird, und wobei dieses Produkt wahlweise zur Herstellung einer pestiziden Zusammensetzung formuliert wird.

2. Verfahren nach Anspruch 1, wobei der mutante oder variante Stamm von Bacillus thuringiensis subsp. tenebrionis durch Mutation und Selektion der Bacillus thuringiensis subsp. tenebrionis Stämme erhalten wird, wobei der Hauptstamm folgenden Schritten unterzogen wird:
(i) Behandlung mit einem Mutagen,
(ii) Aufzucht der so behandelten Mutanten in einem Medium, das für die Selektion von asporogenen und/oder oligosporogenen Stämmen geeignet ist,
(iii) Selektion durchscheinender Kolonien und Aufzucht in einem Medium, das sich bei Erwärmung nicht Verflüssigt, und
(iv) Deselektion echt asporogener Stämme durch Hitzebehandlung der Kolonien.

3. Verfahren nach Anspruch 1 oder 2, wobei die Mutante oder Variante des B. thuringiensis subsp. tenebrionis Stammes zur Herstellung von doppelt so viel insektiziden delta-Endotoxinen wie ihr Urstamm befähigt ist.

4. Verfahren nach Anspruch 1, 2 oder 3, worin die Mutante oder Variante des B. thuringiensis subsp. tenebrionis Stammes zum Pathotyp C von B. thuringiensis subsp. tenebrionis zählt.

5. Verfahren nach Anspruch 1, 2, 3 oder 4, wobei die Mutante oder Variante des B. thuringiensis subsp. tenebrionis Stammes zur Erzeugung von mehr als der dreifachen Menge an delta-Endotoxin wie der Stamm DSM 2803 fähig ist.

6. Verfahren nach Anspruch 4 oder 5, wobei die Mutante oder Variante des B. thuringiensis subsp. tenebrionis Stammes eine Sporulationsfrequenz aufweist, die 10 bis 100 oder gar 10⁶ mal niedriger ist, als die Sporulationsfrequenz des Stammes DSM 2803.

7. Verfahren nach Anspruch 1 oder 2, wobei die Mutante die hinterlegte Mutante des B. thuringiensis subsp. tenebrionis Stammes DSM 5480 ist.

8. Verfahren nach Anspruch 2, wobei die so ausgewählten Kolonien in einem normalen Produktionsmedium aufgezogen werden, und wobei eine Endselektion von Stämmen durchgeführt wird, die zur Erhöhung der delta-Endotoxin-Produktion fähig sind.

9. Verfahren nach Anspruch 2 oder 8, wobei in Schritt (i) das Mutagen jedwedes geeignete chemische Mutagen ist, wie etwa N-Methyl-N'-nitro-N-nitrosoguanidin oder Ethylmethansulfonat.

10. Verfahren nach Anspruch 2 oder 8, worin der Urstamm in Schritt (i) mit einer geeigneten Menge elektromagnetischer Strahlung, wie etwa γ-, oder Röntgen- oder UV-Strahlung behandelt wird.

11. Verfahren nach irgendeinem der Ansprüche 2 und 8-10, wobei in Schritt (ii) das Medium ein verändertes Sporulationsnährmedium ist, das Phosphat umfaßt (NSMP Medium).

12. Verfahren nach irgendeinem der Ansprüche 2 und 8-11, wobei in Schritt (iv) das Medium ein NSMP Medium ist, das mit MgCl₂ und Gelrite, Kelco bezusatzt ist.

13. Verfahren nach Anspruch 1, wobei das Bacillus thuringiensis subsp. tenebrionis delta-Endotoxin-Produkt mit landwirtschaftlich verträglichen Hilfstoffen formuliert wird, unter Erhalt einer pestiziden Zusammensetzung worin eben dieses Produkt den aktiven Bestandteil darstellt.

14. Verfahren nach Anspruch 13, wobei das delta-Endotoxin-Produkt entweder alleine oder in Kombination mit anderen biozid aktiven Produkten zusammen formuliert wird, unter Erzeugung der pestiziden Zusammensetzung.

15. Verfahren nach Anspruch 13 oder 14, wobei das delta-Endotoxin-Produkt mit einem landwirtschaftlich verträglichen Verdünner oder Träger zum Erhalt der pestiziden Zusammensetzung vereinigt wird.

16. Verfahren nach Anspruch 13, 14 oder 15 wobei die pestizide Zusammensetzung eine Stärke hat, die mehr als doppelt so groß ist, wie diejenige der pestiziden Zusammensetzungen, die mit dem Urstamm hergestellt werden.

17. Verfahren nach Anspruch 16, wobei die pestizide Zusammensetzung eine Stärke von wenigstens 15,000 BTTTU/g aufweist, entsprechend wenigstens 3 Gewichtsprozent an gegenüber Coleoptera insektizidem Kristallprotein.

18. Verfahren nach Anspruch 16, wobei die pestizide Zusammensetzung eine Stärke von wenigstens 50,000 BTTTU/g aufweist, entsprechend wenigstens 10 Gewichtsprozent an gegenüber Coleoptera insektizidem Kristallprotein.

19. Verfahren nach Anspruch 7, wobei die pestizide Zusammensetzung wenigstens die doppelte Stärke der pestiziden Zusammensetzungen aufweist, die aus DSM 2803 oder anderen gegen Coleoptera aktiven Btt Stämmen herstellbar sind.

20. Verfahren nach irgendeinem der Ansprüche 13 bis 19 wobei der Verdünner oder Träger in der Zusammensetzung ein Feststoff oder eine Flüssgikeit ist, wahlweise in Zusammenwirkung mit einem oberflächenaktiven Mittel.

21. Verwendung des B. thuringiensis subsp. tenebrionis delta-Endotoxin-Produktes, das in Übereinstimmung mit dem Verfahren nach irgendeinem der Ansprüche 1 und den davon abhängigen Ansprüchen 3-7, erhältlich ist, bei der Herstellung von pestiziden Zusammensetzungen, die dasselbe als aktiven Bestandteil enthalten, zur Anwendung auf Flächen, die mit dem Schädling befallen sind.

22. Verwendung nach Anspruch 21, wobei das delta-Endotoxin-Produkt entweder alleine oder in Kombination mit anderen bioazid aktiven Produkten verwendet wird.

23. Verwendung nach Anspruch 21 oder 22, wobei das delta-Endotoxin-Produkt in Vermengung mit einem landwirtschaftlich vertäglichen Verdünner oder Träger verwendet wird.

24. Verwendung nach Anspruch 21, 22 oder 23 wobei die pestizide Zusammensetzung eine Stärke aufweist, die mehr als doppelt so groß ist, wie diejenige der pestiziden Zusammensetzungen, die mit dem Urstamm hergestellt werden.

25. Verwendung nach Anspruch 23, wobei die pestizide Zusammensetzung eine flüssige pestizide Zusammensetzung ist, die eine Stärke von wenigstens 15,000BTTU/g aufweist, entsprechend wenigstens drei Gewichtsprozent an gegenüber Coleoptera insektizidem Kristallprotein.

26. Verwendung nach Anspruch 23, wobei die pestizide Zusammensetzung eine trockene pestizide Zusammensetzung ist, die eine Stärke von wenigstens 50,000 BTTU/g aufweist, entsprechend wenigstens 10 Gewichtsprozent an gegenüber Coleoptera insektizidem Kristallprotein.

27. Verwendung nach Anspruch 21, wobei das delta-Endotoxin-Produkt aus dem Stamm DSM 5480 erhalten wird.

28. Verwendung nach irgendeinem der Ansprüche 21-27, wobei der Verdünner oder Träger in der Zusammensetzung ein Feststoff oder eine Flüssigkeit ist, wahlweise in Zusammenwirkung mit einem oberflächenaktiven Mittel.

29. Verwendung nach irgendeinem der Ansprüche 21-28, wobei der Schädling zu den Coleoptera zählt, insbesondere der Coloradokartoffelkäfer.

30. Verwendung nach Anspruch 29, wobei die pestizide Zusammensetzung eine flüssige pestizide Zusammensetzung ist, und wobei der Schädling durch die Anwendung von 23,4ml/are (1 quart per acre) der flüssigen pestiziden Zusammensetzung bekämpft werden kann.

31. Verwendung nach Anspruch 29, wobei die pestizide Zusammensetzung eine trockene Pestizidzusammensetzung ist, und wobei der Schädling durch die Anwendung von 5,6gram/are (0,51b. per acre) der trockenen pestiziden Zusammensetzung bekämpft werden kann.

32. Pestizide Zusammensetzung, dadurch gekennzeichnet, daß sie folgendes umfaßt:
(i) 0,5 bis 25 Gewichtsprozent eines delta-Endotoxin-Produktes einer B. thuringiensis subsp. tenebrionis Mutante oder Variante, erhältlich gemäß dem Verfahren nach Anspruch 1; und
(ii) einen landwirtschaftlich verträglichen Hilfsstoff.

33. Pestizide Zusammensetzung nach Anspruch 32, wobei das delta-Endotoxin-Produkt alleine oder in Kombination mit anderen bioazid aktiven Produkten verwendet wird.

34. Pestizide Zusammensetzung nach Anspruch 32 oder 33, wobei das delta-Endotoxin-Produkt in Vermengung mit einem landwirtschaftlich verträglichen Verdünner oder Träger verwendet wird.

35. Pestizide Zusammensetzung nach Anspruch 32, 33 oder 34 mit einer Stärke, die mehr als doppelt so groß wie diejenige der pestiziden Zusammensetzungen ist, die mit dem Urstamm hergestellt werden.

36. Flüssige pestizide Zusammensetzung nach Anspruch 34 mit einer Stärke von wenigstens 15,000 BTTU/g entsprechend wenigstens 3 Gewichtsprozent an gegenüber Coleoptera insektizidem Kristallprotein.

37. Trockene pestizide Zusammensetzung nach Anspruch 34 mit einer Stärke von wenigstens 50,000 BTTU/g, entsprechend wenigstens 10 Gewichtsprozent an gegenüber Coleoptera insektizidem Kristallprotein.

38. Pestizide Zusammensetzung nach Anspruch 32, wobei das delta-Endotoxin-Produkt aus DSM 5480 hergestellt wird, und wenigstens die doppelte Stärke der pestiziden Zusammensetzungen aufweist, die aus DSM 2803 oder anderen gegenüber Coleoptera aktiven Btt Stämmen herstellbar sind.

39. Zusammensetzung nach irgendeinem der Ansprüche 32-37, wobei der Verdünner oder Träger in der Zusammensetzung ein Feststoff oder eine Flüssigkeit ist, wahlweise in Zusammenwirkung mit einem oberflächenaktiven Mittel.

## Revendications (Revendications pour l'(les) Etat(s) contractant(s) suivant(s): AT, BE, CH, LI, DE, DK, FR, GB, GR, IT, LU, NL, SE,)

1. Souche mutante ou variante d'une souche de *Bacillus thuringiensis subsp. tenebrionis* capable de produire de grandes quantités d'endotoxines delta insecticides par comparaison à sa souche mère et ayant un cristal parasporal ayant une longueur d'arête moyenne d'au moins 2 µm et une fréquence de sporulation au moins 10 fois plus faible que la fréquence de sporulation de la souche mère, les endotoxines delta produites par ladite souche mutante ou variante de B. *thuringiensis subsp. tenebrionis* étant actives contre les coléoptères.

2. Souche mutante ou variante selon la revendication 1, dans laquelle la souche de *B. thuringiensis subsp. tenebrionis* mutante ou variante est capable de produire au moins deux fois autant d'endotoxines delta insecticides que sa souche mère.

3. Souche mutante ou variante selon la revendication 1 ou 2, dans laquelle la souche de *B. thuringiensis subsp. tenebrionis* mutante ou variante appartient au pathotype C de *B. thuringiensis subsp. tenebrionis.*

4. Souche mutante ou variante selon la revendication 1, 2 ou 3, dans laquelle la souche de *B. thuringiensis subsp. tenebrionis* mutante ou variante est capable de produire plus de trois fois autant d'endotoxines delta que la souche DSM 2803.

5. Souche mutante ou variante selon la revendication 3 ou 4, dans laquelle la souche de *B. thuringiensis subsp. tenebrionis* mutante ou variante présente une fréquence de sporulation 10 ou 100 ou même 10⁶ fois inférieure à la fréquence de sporulation de la souche DSM 2803.

6. Souche mutante selon la revendication 1, qui est la souche de *B. thuringiensis subsp. tenebrionis* mutante déposée DSM 5480.

7. Procédé de production d'un produit à base d'endotoxines delta de B. *thuringiensis subsp. tenebrionis* à l'aide d'une souche mutante ou variante selon l'une quelconque des revendications 1 à 6, selon lequel on cultive la souche mutante ou variante de *B. thuringiensis* dans un milieu de culture approprié comprenant des sources de carbone, d'azote et d'autres constituants pendant un temps approprié, puis on récupère à partir du milieu de culture un produit insecticide comprenant les endotoxines delta, seul ou en combinaison avec des cellules et/ou des spores, et on mélange éventuellement ce produit avec des excipients acceptables en agriculture.

8. Composition pesticide comprenant comme constituant actif le produit à base d'endotoxines delta de *B. thuringiensis subsp. tenebrionis* pouvant être obtenu selon la revendication 7.

9. Composition pesticide selon la revendication 8, dans laquelle le produit à base d'endotoxines delta est utilisé seul ou en combinaison avec d'autres agents à activité biocide.

10. Composition pesticide ou préparation selon la revendication 8 ou 9, comprenant le produit à base d'endotoxines delta de *B. thuringiensis subsp. tenebrionis* en mélange avec un diluant ou un support acceptable en agriculture.

11. Composition pesticide selon la revendication 8, 9 ou 10, qui a une activité de plus de deux fois celle des compositions pesticides produites avec la souche mère.

12. Composition pesticide liquide selon la revendication 10, qui a une activité d'au moins 15 000 BTTU/g, correspondant à au moins 3 % en masse de protéine cristal insecticide pour les coléoptères.

13. Composition pesticide sèche selon la revendication 10, qui a une activité d'au moins 50 000 BTTU/g, correspondant à au moins 10 % en masse de protéine cristal insecticide pour les coléoptères.

14. Composition pesticide produite par la souche DSM 5480, ayant au moins deux fois l'activité des compositions pesticides produites par la souche DSM 2803 ou d'autres souches de *Btt* actives contre les coléoptères.

15. Composition selon l'une quelconque des revendications 8 à 14, dans laquelle le diluant ou le support de la composition est un solide ou un liquide éventuellement en combinaison avec un agent tensioactif.

16. Procédé de lutte contre les insectes nuisibles, selon lequel on applique une composition pesticide selon l'une quelconque des revendications 8 à 15 à une zone infestée par ledit insecte nuisible.

17. Procédé selon la revendication 16, dans lequel l'insecte nuisible est un coléoptère, en particulier le doryphore.

18. Procédé selon la revendication 17, dans lequel ladite composition pesticide est une composition pesticide liquide et dans lequel l'insecte nuisible peut être détruit par l'application de 23,4 ml/are (1 quart par acre) de ladite composition pesticide liquide.

19. Procédé selon la revendication 17, dans lequel ladite composition pesticide est une composition pesticide sèche et dans lequel l'insecte nuisible peut être détruit par l'application de 5,6 g/are (0,5 lb par acre) de ladite composition pesticide sèche.

20. Procédé de mutation de souches de *Bacillus thuringiensis subsp. tenebrionis* et de sélection des mutants selon la revendication 1 capables de produire des quantités d'endotoxines delta essentiellement plus grandes que leurs souches mères, dans lequel :
i) on traite la souche mère avec un agent mutagène,
ii) on cultive les mutants ainsi traités dans un milieu approprié pour la sélection de souches asporogènes et/ou oligosporogènes,
iii) on sélectionne les colonies translucides et on les cultive dans un milieu qui ne se liquéfie pas au chauffage, et
iv) on élimine de la sélection les souches réellement asporogènes en soumettant les colonies à un traitement thermique.

21. Procédé selon la revendication 20, dans lequel on cultive les colonies ainsi sélectionnées dans un milieu de production normal, et on effectue une sélection finale des souches capables d'augmenter la production d'endotoxines delta.

22. Procédé selon la revendication 20 ou 21, dans lequel, dans l'étape (i), l'agent mutagène est un agent mutagène chimique approprié quelconque comme la N-méthyl-N'-nitro-N-nitrosoguanidine, ou le méthanesulfonate d'éthyle.

23. Procédé selon la revendication 20 ou 21, dans lequel on traite la souche mère dans l'étape (i) avec un rayonnement électromagnétique approprié comme des rayons γ, X ou UV.

24. Procédé selon l'une quelconque des revendications 20 à 23, dans lequel, dans l'étape (ii), ledit milieu est un milieu de sporulation nutritif modifié comprenant du phosphate (milieu NSMP).

25. Procédé selon l'une quelconque des revendications 20 à 24, dans lequel, dans l'étape (iv), ledit milieu est un milieu NSMP complété avec du MgCl₂ et du Gelrite, Kelco.

## Revendications (Revendications pour l'(les) Etat(s) contractant(s) suivant(s): ES)

1. Procédé de production d'un produit à base d'endotoxines delta de *B.acillus thuringiensis subsp. tenebrionis* à l'aide d'une souche mutante ou variante d'une souche de *B. thuringiensis subsp. tenebrionis* capable de produire de grandes quantités d'endotoxines delta insecticides par comparaison à sa souche mère et ayant un cristal parasporal ayant une longueur d'arête moyenne d'au moins 2 µm et une fréquence de sporulation au moins 10 fois plus faible que la fréquence de sporulation de la souche mère, les endotoxines delta produites par ladite souche mutante ou variante de *B. thuringiensis subsp. tenebrionis* étant actives contre les coléoptères, ledit procédé comprenant la culture de ladite souche mutante ou variante de *B. thuringiensis subsp. tenebrionis* dans un milieu de culture approprié comprenant des sources de carbone, d'azote et d'autres constituants pendant un temps approprié, puis la récupération à partir du milieu de culture d'un produit insecticide comprenant les endotoxines delta, seul ou en combinaison avec des cellules et/ou des spores, et éventuellement la formulation de ce produit pour la préparation d'une composition pesticide.

2. Procédé selon la revendication 1, dans lequel la souche mutante ou variante de *Bacillus thuringiensis subsp. tenebrionis* est obtenue par mutation et sélection de souches de *Bacillus thuringiensis subsp. tenebrionis,* selon lesquelles
i) on traite la souche mère avec un agent mutagène,
ii) on cultive les mutants ainsi traités dans un milieu approprié pour la sélection de souches asporogènes et/ou oligosporogènes,
iii) on sélectionne les colonies translucides et on les cultive dans un milieu qui ne se liquéfie pas au chauffage, et
iv) on élimine de la sélection les souches réellement asporogènes en soumettant les colonies à un traitement thermique.

3. Procédé selon la revendication 1 ou 2, dans lequel la souche de *B. thuringiensis subsp. tenebrionis* mutante ou variante est capable de produire au moins deux fois autant d'endotoxines delta insecticides que sa souche mère.

4. Procédé selon la revendication 1, 2 ou 3, dans lequel la souche de *B. thuringiensis subsp. tenebrionis* mutante ou variante appartient au pathotype C de *B. thuringiensis subsp. tenebrionis.*

5. Procédé selon la revendication 1, 2, 3 ou 4, dans lequel la souche de *B.* *thuringiensis subsp. tenebrionis* mutante ou variante est capable de produire plus de trois fois autant d'endotoxines delta que la soucheDSM 2803.

6. Procédé selon la revendication 4 ou 5, dans lequel la souche de *B. thuringiensis subsp. tenebrionis* mutante ou variante présente une fréquence de sporulation 10 ou 100 ou même 10⁶ fois inférieure à la fréquence de sporulation de la souche DSM 2803.

7. Procédé selon la revendication 1 ou 2, dans lequel la souche mutante est la souche de *B. thuringiensis subsp. tenebrionis* mutante déposée DSM 5480.

8. Procédé selon la revendication 2, dans lequel on cultive les colonies ainsi sélectionnées dans un milieu de production normal, et on effectue une sélection finale des souches capables d'augmenter la production d'endotoxines delta.

9. Procédé selon la revendication 2 ou 8, dans lequel, dans l'étape (i), l'agent mutagène est un agent mutagène chimique approprié quelconque comme la N-méthyl-N'-nitro-N-nitrosoguanidine, ou le méthanesulfonate d'éthyle.

10. Procédé selon la revendication 2 ou 8, dans lequel on traite la souche mère dans l'étape (i) avec un rayonnement électromagnétique approprié comme des rayons γ, X ou UV.

11. Procédé selon l'une quelconque des revendications 2 et 8 à 10, dans lequel, dans l'étape (ii), ledit milieu est un milieu de sporulation nutritif modifié comprenant du phosphate (milieu NSMP).

12. Procédé selon l'une quelconque des revendications 2 et 8 à 11, dans lequel, dans l'étape (iv), ledit milieu est un milieu NSMP complété avec du MgCl₂ et du Gelrite, Kelco.

13. Procédé selon la revendication 1, dans lequel on formule le produit à base d'endotoxines delta de *B. thuringiensis subsp. tenebrionis* avec des excipients acceptables en agriculture pour obtenir une composition pesticide dans laquelle ledit produit constitue l'ingrédient actif.

14. Procédé selon la revendication 13, dans lequel on formule ledit produit à base d'endotoxines delta seul ou en combinaison avec d'autres produits à activité biocide pour produire la composition pesticide.

15. Procédé selon la revendication 13 ou 14, dans lequel on combine le produit à base d'endotoxines delta avec un diluant ou un support acceptable en agriculture pour obtenir la composition pesticide.

16. Procédé selon la revendication 13, 14 ou 15, dans lequel la composition pesticide a une activité de plus de deux fois celle des compositions pesticides produites avec la souche mère.

17. Procédé selon la revendication 16, dans lequel la composition pesticide a une activité d'au moins 15 000 BTTU/g, correspondant à au moins 3 % en masse de protéine cristal insecticide pour les coléoptères.

18. Procédé selon la revendication 16, dans lequel la composition pesticide a une activité d'au moins 50 000 BTTU/g, correspondant à au moins 10 % en masse de protéine cristal insecticide pour les coléoptères.

19. Procédé selon la revendication 7, dans lequel la composition pesticide a au moins deux fois l'activité des compositions pesticides produites par la souche DSM 2803 ou d'autres souches de *Btt* actives contre les coléoptères.

20. Procédé selon l'une quelconque des revendications 13 à 19, dans lequel le diluant ou le support de la composition est un solide ou un liquide éventuellement en combinaison avec un agent tensioactif.

21. Utilisation du produit à base d'endotoxines delta de *B. thuringiensis subsp. tenebrionis,* pouvant être obtenu selon le procédé de l'une quelconque de la revendication 1 et des revendications 3 à 7 qui en dépendent, dans la production de compositions pesticides qui le contiennent en tant que constituant actif, destinées à l'application sur une zone infestée par ledit insecte nuisible.

22. Utilisation selon la revendication 21, dans laquelle le produit à base d'endotoxines delta est utilisé seul ou en combinaison avec d'autres produits à activité biocide.

23. Utilisation selon la revendication 21 ou 22, dans laquelle le produit à base d'endotoxines delta est utilisé en mélange avec un diluant ou un support acceptable en agriculture.

24. Utilisation selon la revendication 21, 22 ou 23, dans laquelle la composition pesticide a une activité de plus de deux fois celle des compositions pesticides produites avec la souche mère.

25. Utilisation selon la revendication 23, dans laquelle la composition pesticide est une composition pesticide liquide ayant une activité d'au moins 15 000 BTTU/g, correspondant à au moins 3 % en masse de protéine cristal insecticide pour les coléoptères.

26. Utilisation selon la revendication 23, dans laquelle la composition pesticide est une composition pesticide sèche ayant une activité d'au moins 50 000 BTTU/g, correspondant à au moins 10 % en masse de protéine cristal insecticide pour les coléoptères.

27. Utilisation selon la revendication 21, dans laquelle le produit à base d'endotoxines delta est produit par la souche DSM 5480.

28. Utilisation selon l'une quelconque des revendications 21 à 27, dans laquelle le diluant ou le support de la composition est un solide ou un liquide éventuellement en combinaison avec un agent tensioactif.

29. Utilisation selon l'une quelconque des revendications 21 à 28, dans laquelle l'insecte nuisible est un coléoptère, en particulier le doryphore.

30. Utilisation selon la revendication 29, dans laquelle ladite composition pesticide est une composition pesticide liquide et dans laquelle l'insecte nuisible peut être détruit par l'application de 23,4 ml/are (1 quart par acre) de ladite composition pesticide liquide.

31. Utilisation selon la revendication 29, dans laquelle ladite composition pesticide est une composition pesticide sèche et dans laquelle l'insecte nuisible peut être détruit par l'application de 5,6 g/are (0,5 lb par acre) de ladite composition pesticide sèche.

32. Composition pesticide, caractérisée en ce qu'elle comprend
(i) 0,5 à 25 % en masse d'un produit à base d'endotoxines delta d'une souche mutante ou variante de *B. thuringiensis subsp. tenebrionis,* obtenu par le procédé de la revendication 1 ; et
(ii) un excipient acceptable en agriculture.

33. Composition pesticide selon la revendication 32, dans laquelle le produit à base d'endotoxines delta est utilisé seul ou en combinaison avec d'autres produits à activité biocide.

34. Composition pesticide selon la revendication 32 ou 33, dans laquelle le produit à base d'endotoxines delta est utilisé en mélange avec un diluant ou un support acceptable en agriculture.

35. Composition pesticide selon la revendication 32, 33 ou 34, qui a une activité de plus de deux fois celle des compositions pesticides produites avec la souche mère.

36. Composition pesticide liquide selon la revendication 34, ayant une activité d'au moins 15 000 BTTU/g, correspondant à au moins 3 % en masse de protéine cristal insecticide pour les coléoptères.

37. Composition pesticide sèche selon la revendication 34, ayant une activité d'au moins 50 000 BTTU/g, correspondant à au moins 10 % en masse de protéine cristal insecticide pour les coléoptères.

38. Composition pesticide selon la revendication 32, dans laquelle le produit à base d'endotoxines delta est produit par la souche DSM 5480, ayant au moins deux fois l'activité des compositions pesticides produites par la souche DSM 2803 ou d'autres souches de *Btt* actives contre les coléoptères.

39. Composition selon l'une quelconque des revendications 32 à 37, dans laquelle le diluant ou le support de la composition est un solide ou un liquide éventuellement en combinaison avec un agent tensioactif.
